# EUROPÄISCHE PATENTSCHRIFT

(11) **EP 0 493 698 B1**
(45) Veröffentlichungstag und Bekanntmachung des Hinweises auf die Patenterteilung: **05.04.1995**
(21) Anmeldenummer: 91120802.3
(22) Anmeldetag: 04.12.1991
(51) Int. Cl.: A61F 2/44, A61F 2/28

(54) **Knochenersatz**
Bone replacement
Prothèse osseuse

(30) Priorität: 19.12.1990 DE 4040581; 19.10.1991 DE 4101526
(43) Veröffentlichungstag der Anmeldung: 08.07.1992
(60) Teilanmeldung: 94110049.7
(73) Patentinhaber: Härle, Anton, Prof. Dr., D-48161 Münster (DE)
(72) Erfinder: Härle, Anton, Prof. Dr., D-48161 Münster (DE)
(74) Vertreter: Habbel, Hans-Georg, Dipl.-Ing.

(56) Entgegenhaltungen:
- EP-A- 0 353 476
- WO-A-87/06842
- WO-A-87/07827
- WO-A-88/01517
- WO-A-89/09035
- WO-A-90/00037
- US-A- 4 643 735

## Beschreibung

Die Erfindung bezieht sich auf einen Knochenersatz gemäß dem Oberbegriff des Hauptanspruches.

Die rekonstruktiven, medizinischen Behandlungsverfahren erfordern in zunehmendem Maße Knochenersatzmaterialien, um die formgerechte Wiederherstellung bzw. den Aufbau einer anatomischen Form von Knochen und Knochenanteilen erreichen zu können, da die Verwendung von körpereigenem Knochengewebe häufig an die Grenze der ausreichenden Verfügbarkeit stößt und auch mit der Problematik der Zweitkrankheit am Entnahmeort belastet ist.

Der Einsatz von der Knochengrundsubstanz ahnlichen Hydroxylapatit-Keramikmaterial hat dabei in den letzten 20 Jahren nicht zu dem erwarteten Erfolg geführt. Zwar ist es gelungen, sehr verschiedene Biokeramikmaterialien künstlich mit unterschiedlicher Löslichkeit und Resorptionseigenschaften herzustellen, doch konnten bisher mit Ausnahme weniger Anwendungsgebiete Hydroxylapatitkeramiken die autologe Knochentransplantation nicht ersetzen, nicht einmal mengenmäßig einschränken.

Die Gründe dafür liegen darin, daß die bisherigen Biokeramik-Materialien entweder keine ausreichende Stabilität im Sinne einer mechanischen Beanspruchbarkeit aufweisen oder, wenn sie diese Eigenschaft haben, nicht in Körpergewebe integriert werden, d. h. eine unzureichende Biodegradibilität besitzen.

Sollen Knochenersatzmaterialien aber ihre mechanische Aufgabe erfüllen, müssen sie zuerst mit dem vorhandenen Knochen eine innige und belastbare Verbindung eingehen, was nur über eine teilweise Auflösung und Knochenneubildung an der Oberfläche des Ersatzgewebes erfolgen kann. Während also einerseits die biologische Abbaubarkeit eine Grundvoraussetzung darstellt, damit Knochenersatzgewebe in den körpereigenen Knochen integriert werden kann, was also eine hohe Biodegradibilität erfordert, wird vom Knochenersatzgewebe andererseits eine mechanische Belastbarkeit erwartet, die mindestens solange bestehen muß, bis durch Integration und Knochenneubildung das Stabilitätsdefizit beseitigt ist. Diese beiden Anforderungen schließen sich aber gegenseitig in einer Materialkomponente nahezu aus.

Aus der WO 90-00037 ist ein künstliches Wirbelsäulenimplantat vorgeschlagen worden, das härter als Knochen sein soll. Das Implantat soll Makrozellen und Öffnungen aufweisen, die mit körpereigenem Knochen ausgefüllt werden können, um damit das Implantat mit den übrigen Knochenbereichen zu verbinden. Über den Werkstoff dieses Implantates wird in der Literaturstelle nichts ausgeführt.

Auch in der US-A 46 43 735 wird ein Ersatzmaterial in Verbindung mit Knochen vorgeschlagen, wobei dieses Ersatzmaterial porös sein soll, um damit dem verbleibenden Knochen ein Einwachsen in dieses Material zu ermöglichen. Die Porösität soll so gewählt werden, daß sie vorzugsweise zwischen 150 - 750 m²/g liegt.

Schließlich ist aus der WO 87/07827 ein Implantat zum Fixieren benachbarter Wirbelknochen der Wirbelsäule bekanntgeworden, das aus einem zylindrischen oder rohrförmigen offenzelligen metallischen Körper besteht, der keilförmig ausgebildet ist und an seinem proximalen Ende massiv ausgebildet wird, um somit eine stabile Fläche für ein Einschlagwerkzeug zu bilden.

Demgegenüber liegt der Erfindung die Aufgabe zugrunde, einen Knochenersatz zu schaffen, der eine gute Biodegradibilität mit einer hohen mechanischen Belastbarkeit verbindet.

Diese der Erfindung zugrundeliegende Aufgabe wird, ausgehend von der Lehre gemäß dem Oberbegriff des Hauptanspruches, durch die Lehre des kennzeichnenden Teiles des Hauptanspruches gelöst.

Mit anderen Worten ausgedrückt wird vorgeschlagen, daß z. B. ein biokeramischer Knochenersatz geschaffen wird, der aus einer Kombination verschiedener Materialien in einem Verbund besteht, wobei die mechanische Belastbarkeit und Formkonstanz von einer ersten Materialkomponente "Festigkeit" gewährleistet wird, während die biologische Integration und Anregung zur Knochenneubildung und biomechanische Funktions- und Formanpassung über eine zweite Materialkomponente "Integration" angestrebt wird. Die von der zweiten Materialkomponente "Integration" ausgehende Wirkung zur biologischen Integration erfolgt in einem mechanisch stabilisierten Formrahmen und in einer Raumausdehnung, die durch die erste Materialkomponente "Festigkeit" gesichert und vorgegeben ist.

Die knöcherne Integration läuft daher an der Oberfläche der ersten Materialkomponente "Festigkeit" ab, ohne diese zu ersetzen oder aufzulösen, wobei der die spätere biomechanische Funktion leistende, neu gebildete Knochen bzw. die knöcherne Verbindung durch die zweite Materialkomponente "Integration" induziert wird und an ihr durch völligen oder partiellen Abbau abläuft.

Die schwer abbaubare erste Materialkomponente "Festigkeit" erbringt in dieser Übergangsphase die mechanische Stabilität und wird so gleichsam zu einer Matrix, auf der das neu gebildete Knochengewebe bzw. der biologische Intergrationsprozeß der zweiten Material komponente "Integration" abläuft.

Damit der Einsatz derartiger biokeramischer Knochenersatzbauteile in optimierter Weise durchgeführt werden kann, müssen die Materialkomponenten in den erfindungsgemäßen Ausgestaltungen vorliegen. So darf z. B. die mechanische Aufgabenbewältigung der ersten Materialkomponente "Festigkeit" nicht die innige, biologische Integration behindern. Dies erfordert aber, daß zwischen dem ortsständigen Knochen und der zweiten Materialkomponente "Integration" ein direkter Kontakt durch ausreichend große Öffnungen in der ersten Materialkomponente "Festigkeit" ermöglicht wird. Wegen der schon primär sehr hohen Sprödigkeit der Biokeramikwerkstoffe, insbesondere von statisch belastbaren Werkstoffen, droht bei Materialschwächung durch Öffnungen eine erhöhte Bruchgefahr in dem Bauteil mit der ersten Materialkomponente "Festigkeit", was wiederum eine entsprechende Abstimmung in den Größenverhältnissen, dem Anteil der Materialkomponenten sowie besonderen geometrischen Ausgestaltungen notwendig machen.

Als Materialkomponente "Integration" kann auch Knochengewebe, vorzugsweise körpereigenes Knochengewebe, eingesetzt werden.

Gemäß einem weiteren vorteilhaften Vorschlag entsprechend der Erfindung wird der Formkörper aus porösem Material hergestellt und evakuiert und im evakuierten Zustand vakuumverpackt, so daß dieser unter Vakuum stehende evakuierte Formkörper an den Arzt ausgeliefert wird. Der Arzt kann nunmehr in die diesen evakuierten Formkörper enthaltende Verpackung ein oder mehrere Pharmaka einbringen, die von den Poren des Formkörpers aufgenommen werden und dann nach Implantation des Formkörpers in den menschlichen Körper freigesetzt werden.

Vorzugsweise weist der Formkörper eine stumpfe Keilform auf, wobei eine Fläche um einen Winkel α = 2 bis 5° geneigt ist. Hierdurch wird ein Formkörper geschaffen, der in den Zwischenwirbelraum der Wirbelkörper eingesetzt werden kann, wobei die kleine Fläche nach hinten geneigt ist, so daß der Formkörper eingespannt und festgesetzt wird.

Zur Verdeutlichung des Erfindungsgedankens werden in der Zeichnung zwei Formkörper dargestellt, die unterschiedlich hinsichtlich ihrer Aufnahmeöffnungen ausgebildet sind.

Die Zeichnung zeigt in
- Fig. 1: in Draufsicht, eine Ansicht von unten und im Schnitt einen erfindungsgemäßen Körper, in
- Fig. 2: ebenfalls in Draufsicht in Ansicht von unten und in einem Schnitt eine abgewandelte Ausführungsform, in
- Fig. 3: eine weitere abgewandelte Ausführungsform, in
- Fig. 4: den in Fig. 1 dargestellten Körper, wobei die Hohlräume mit der zweiten Materialkomponente ausgefüllt sind, in
- Fig. 5: den in Fig. 2 dargestellten Körper mit ausgefüllten Hohlräumen und in
- Fig. 6: den Körper gemäß Fig. 3 mit ausgefülltem Hohlraum.

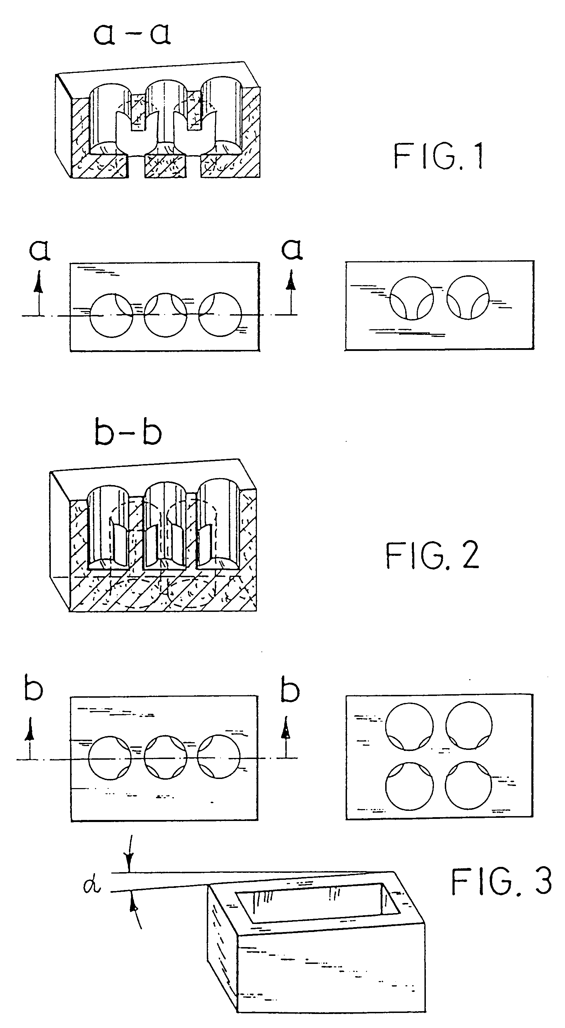

Die in den Fig. 1 und 2 dargestellten Formkörper sind leicht keilförmig mit einem Neigungswinkel α von 2 bis 5° ausgebildet und bestehen aus einer Mischung aus zwei Materialkomponenten, von denen die erste Materialkomponente eine hohe mechanische Festigkeit und die zweite Materialkomponente eine gute biologische Integration bzw. hohe Knochenkonduktivität besitzt.

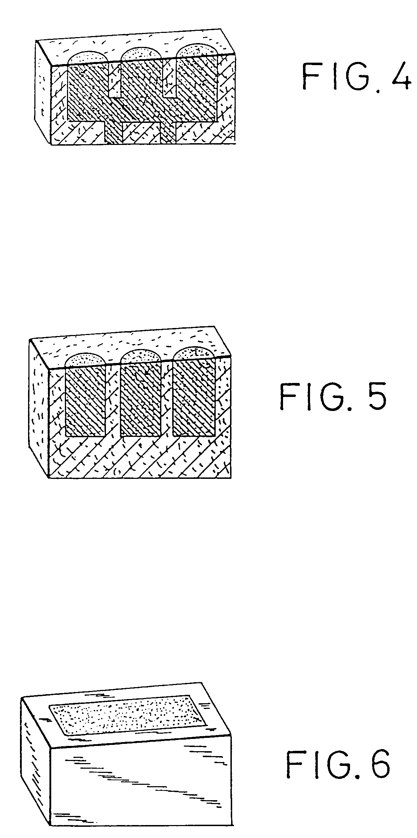

In den Fig. 1 bis 3 sind die aus der ersten Materialkomponente bestehenden Körper und in den Fig. 4 bis 6 sind diese Körper, angefüllt mit der zweiten Materialkomponente, dargestellt.

Die Zeichnungen verdeutlichen die Möglichkeit des Anbringens der Öffnungen, wobei die Seitenflächen von Öffnungen frei sind. Hierdurch wird zwar eine gute Durchdringbarkeit, aber eine ausreichende Festigkeit erreicht.

Fig. 3 zeigt eine Ausführungsform, bei welcher nur ein Randbereich des Körpers aus der ersten Materialkomponente "Festigkeit" gebildet wird, während der innere Bereich offen ist, wobei in diesen inneren Bereich Knochengewebe, vorzugsweise körpereigenes Knochengewebe, eingesetzt werden kann, das dann die Materialkomponente "Integration" bildet. Dies ist in Fig. 6 dargestellt.

Aus der Zeichnung nicht erkennbar, aber im Rahmen der Erfindung liegend, ist vorgesehen, daß der den Formkörper bildende Werkstoff porös ausgebildet ist.

Wird ein solcher poröser Formkörper evakuiert und im evakuierten Zustand entsprechend verpackt, z. B. an einen Operateur ausgeliefert, ist es möglich, nunmehr durch Injektion in die unter Vakuum stehende Verpackung in den porösen Formkörper "Pharmaka" einzuspritzen, die begierig von den Poren aufgenommen werden, so daß dann, wenn dieser Formkörper implantiert wird, im menschlichen Körper diese Pharmaka freigegeben und freigesetzt werden können.

Durch die aus den Zeichnungen ersichtliche Keilform des Formkörpers wird ein idealer Formkörper für Wirbelkörperverblockungen geschaffen, d. h. der Körper kann in den Zwischenwirbelraum eingesetzt werden, wobei die kleine Fläche nach hinten gerichtet ist, so daß dadurch ein automatisches Festsetzen des Formkörpers erfolgt.

## Patentansprüche

1. Knochenersatz zur Verwendung für Knochendefektauffüllungen und/oder Knochendistraktionen im oder am menschlichen Körper in Form von Formkörpern, die aus zwei Materialkomponenten aufgebaut sind, von denen die erste Materialkomponente "Festigkeit" Öffnungen aufweist, während die zweite Materialkomponente "Integration" der Förderung der osteokonduktiven Wirkung dient, dadurch gekennzeichnet, daß die erste Materialkomponente "Festigkeit" aus einem schwer biodegradablem Material besteht und eine mechanisch-statische Beanspruchbarkeit zumindest in einer Achse von größer als 1.000 N/cm² gewährleistet, während die zweite Materialkomponente "Integration" eine spezifische Oberfläche über 1,5 m²/g aufweist.

2. Knochenersatz nach Anspruch 1, dadurch gekennzeichnet, daß die erste Materialkomponente "Festigkeit" aus einem biokeramischen Material mit einer Makroporosität von 1 - 5 mm Porenweite besteht.

3. Knochenersatz nach Anspruch 1, dadurch gekennzeichnet, daß die zweite Materialkomponente "Integration" aus einem biokeramischen Material mit mittlerer Biodegradibilität und einer Mikroporosität von 10 bis 300 » besteht.

4. Knochenersatz nach Anspruch 1 oder 3, dadurch gekennzeichnet, daß die Materialkomponente "Integration" aus Knochengewebe besteht.

5. Knochenersatz nach Anspruch 4, dadurch gekennzeichnet, daß die zweite Materialkomponente "Integration" aus körpereigenem Knochengewebe besteht.

6. Knochenersatz nach einem oder mehreren der vorhergehenden Ansprüche, dadurch gekennzeichnet, daß er eine Wabenstruktur aufweist.

7. Knochenersatz nach Anspruch 6, dadurch gekennzeichnet, daß er eine interkonnektierende Mikrostruktur aufweist, so daß die einzelnen Waben durch Poren miteinander verbunden sind.

8. Knochenersatz nach Anspruch 1, dadurch gekennzeichnet, daß er bei einem minimalen Materialeinsatz zur Erzielung einer gerichteten Belastbarkeit eine Makrostruktur eines Röhren- oder Wabensystems aufweist.

9. Knochenersatz nach Anspruch 8, gekennzeichnet durch offene Querverbindungen der Röhrensysteme.

10. Knochenersatz nach einem oder mehreren der vorhergehenden Ansprüche, dadurch gekennzeichnet, daß in die Formkörper Fixationselemente zum Zusammenwirken mit Stiften und/oder Schrauben vorgesehen sind.

11. Knochenersatz nach einem oder mehreren der vorhergehenden Ansprüche, dadurch gekennzeichnet, daß in dem Formkörper gleichmäßig verteilt Pharmaka, z. B. Antibiotika, Zytostatika oder Radioisotope angeordnet sind, die nach Implantation des Formkörpers freigegeben werden.

12. Knochenersatz nach einem oder mehreren der vorhergehenden Ansprüche, dadurch gekennzeichnet, daß die Formkörper aus miteinander verkoppelungsfähigen Bauteilen aufgebaut sind.

13. Knochenersatz nach einem oder mehreren der vorhergehenden Ansprüche, dadurch gekennzeichnet, daß die Formkörper eine stumpfe Keilform aufweisen, wobei der Keilwinkel α etwa 2 bis 5° beträgt.

14. Knochenersatz nach Anspruch 11, dadurch gekennzeichnet, daß der Formkörper porös ausgebildet ist und im evakuierten Zustand vakuumverpackt zur Verfügung gestellt ist.

15. Verfahren zur Herstellung eines biokeramischen Knochenersatzes, der gemäß einem oder mehreren der vorhergehenden Ansprüche ausgebildet ist, dadurch gekennzeichnet, daß die den biokeramischen Knochenersatz bildenden beiden Komponenten erst zum Zeitpunkt der Verwendung vermischt werden und daß die zweite Materialkomponente "Integration" aus einzelnen Granula mit einer Partikelgröße von 0,1 - 3 mm besteht, wobei der zweiten Materialkomponente "Integration" eine Substanz aus osteoinduktiven Stoffen zugemischt ist.

## Claims

1. A bone substitute for use for bone defect filling and/or bone distraction in or on the human body in the form of shaped members, which are composed of two material components, the first "strength" of which material components comprises apertures while the second material component "integration" serves to promote the osteoconductive effect, characterized in that the first material component "strength" consists of a material of low biodegradability and ensures mechanical/static loadability at least along one axis of more than 1,000 N/cm², while the second material component "integration" has a specific surface of greater than 1.5 m²/g.

2. A bone substitute according to claim 1, characterized in that the first material component "strength" consists of a bioceramic material with a macroporosity of from 1 - 5 mm pore width.

3. A bone substitute according to claim 1, characterized in that the second material component "integration" consists of a bioceramic material with average biodegradability and a microporosity of from 10 to 300 ».

4. A bone substitute according to claim 1 or claim 3, characterized in that the material component "integration" consists of bone tissue.

5. A bone substitute according to claim 4, characterized in that the second material component "integration" consists of the body's own bone tissue.

6. A bone substitute according to any one of the preceding claims, characterized in that it comprises a honeycomb structure.

7. A bone substitute according to claim 6, characterized in that it comprises an interconnecting microstructure, such that the individual honeycomb elements are connected together by pores.

8. A bone substitute according to claim 1, characterized in that it comprises a macrostructure of a tube or honeycomb system to achieve directional loadability with minimal material use.

9. A bone substitute according to claim 8, characterized by open transverse connections of the tube systems.

10. A bone substitute according to any one of the preceding claims, characterized in that fixing elements are provided in the shaped members for interaction with pins and/or screws.

11. A bone substitute according to any one of the preceding claims, characterized in that pharmaceuticals, e.g. antibiotics, cytostatic drugs or radioisotopes are arranged evenly distributed in the shaped members, which pharmaceuticals are released after implantation of the shaped member.

12. A bone substitute according to any one of the preceding claims, characterized in that the shaped members are constructed of components capable of coupling together.

13. A bone substitute according to any one of the preceding claims, characterized in that the shaped members have a truncated wedge shape, the wedge angle α amounting to approximately 2 to 5°.

14. A bone substitute according to claim 11, characterized in that the shaped member is of porous construction and is made available vacuum-packed in the evacuated state.

15. A process for manufacturing a bioceramic bone substitute, which is constructed according to any one of the preceding claims, characterized in that the two components forming the bioceramic bone substitute are not mixed until the point of use and in that the second material component "integration" consists of individual granules with a particle size of from 0.1 - 3 mm, a substance of osteoinductive matter being admixed with the second material component "integration".

## Revendications

1. Prothèse osseuse, à utiliser pour des remplissages de lacunes osseuses et/ou des ostéoplasties par étirement, dans ou sur le corps humain, ayant la forme de corps façonnés formés de deux composants, dont le premier composant "résistance" présente des orifices, alors que le second composant "intégration" répond aux exigences de l'effet stimulateur d'ossification, caractérisé en ce que le premier composant "résistance" est un matériau difficilement biodégradable et assure une résistance mécanique-statique, au moins dans un axe, de plus de 1000 N/cm², alors que le deuxième composant "intégration" a une surface spécifique de plus de 1,5 m²/g.

2. Prothèse osseuse selon la revendication 1, caractérisée en ce que le premier composant "résistance" est fabriqué dans un matériau biocéramique présentant une macroporosité de 1 à 5 mm de largeur des pores.

3. Prothèse osseuse selon la revendication 1, caractérisée en ce que le second composant "intégration" est fabriqué dans un matériau biocéramique à capacité de biodégradation moyenne et ayant une microporosité de 10 à 300 ».

4. Prothèse osseuse selon la revendication 1 ou 3, caractérisée en ce que le composant "intégration" est du tissu osseux.

5. Prothèse osseuse selon la revendication 4, caractérisée en ce que le second composant "intégration" est du tissu osseux du propre corps.

6. Prothèse osseuse selon l'une ou plusieurs des revendications précédentes, caractérisée en ce qu'elle présente une structure alvéolaire.

7. Prothèse osseuse selon la revendication 6, caractérisée en ce qu'elle présente une microstructure à interconnexions, de sorte que les alvéoles sont reliées entre elles par des pores.

8. Prothèse osseuse selon la revendication 1, caractérisée en ce que, pour une utilisation minimale de matériau permettant d'obtenir une résistance ajustée, elle présente une macrostructure d'un système tubulaire ou alvéolaire.

9. Prothèse osseuse selon la revendication 8, caractérisée par des liaisons transversales ouvertes des systèmes tubulaires.

10. Prothèse osseuse selon l'une ou plusieurs des revendications précédentes, caractérisée en ce que des éléments de fixation sont prévus dans les corps façonnés dans le but de coopérer avec des tiges et/ou des vis.

11. Prothèse osseuse selon l'une ou plusieurs des revendications précédentes, caractérisée en ce que l'on répartit de manière régulière dans le corps façonné des produits pharmaceutiques, par exemple des antibiotiques, des cytostatiques ou des radio-isotopes, qui seront libérés après implantation du corps façonné.

12. Prothèse osseuse selon l'une ou plusieurs des revendications précédentes, caractérisée en ce que les corps façonnés sont fabriqués à partir de pièces pouvant s'accoupler entre elles.

13. Prothèse osseuse selon l'une ou plusieurs des revendications précédentes, caractérisée en ce que les corps façonnés ont la forme d'un trapèze tronqué, l'angle α du trapèze se situant à peu près entre 2 et 5°.

14. Prothèse osseuse selon la revendication 11, caractérisée en ce que le corps façonné est poreux et disponible sous une forme dans laquelle on a fait le vide dans un emballage sous vide.

15. Procédé de fabrication d'une prothèse osseuse biocéramique conforme à l'une ou plusieurs des revendications précédentes, caractérisé en ce que les deux composants qui forment la prothèse osseuse biocéramique ne sont mélangés qu'au moment de l'utilisation et que le deuxième composant "intégration" est formé de granulés à taille de grains de 0,1 à 3 mm, une substance à base d'éléments stimulant l'ossification étant mélangée au deuxième composant "intégration".
